(19) Europäisches Patentamt
European Patent Office
Office européen des brevets

(11) EP 0 608 707 B1

(12) **EUROPÄISCHE PATENTSCHRIFT**

(45) Veröffentlichungstag und Bekanntmachung des Hinweises auf die Patenterteilung:
**23.04.1997 Patentblatt 1997/17**

(51) Int. Cl.$^6$: **C07C 2/34**

(21) Anmeldenummer: **94100352.7**

(22) Anmeldetag: **12.01.1994**

(54) **Cooligomere aus alpha-Olefinen und geringen Mengen an Ethylen**
Co-oligomer of alpha olefins and small amounts of ethylene
Co-oligomère d'alpha-oléfines et de faible quantité d'éthylène

(84) Benannte Vertragsstaaten:
**BE DE ES FR GB NL**

(30) Priorität: **28.01.1993 DE 4302254**

(43) Veröffentlichungstag der Anmeldung:
**03.08.1994 Patentblatt 1994/31**

(73) Patentinhaber: **BASF Aktiengesellschaft**
**67063 Ludwigshafen (DE)**

(72) Erfinder:
- **Marczinke, Bernd Lothar, Dr.**
  **D-67346 Speyer (DE)**
- **Mueller, Hans-Joachim, Dr.**
  **D-67269 Gruenstadt (DE)**
- **Kerth, Juergen, Dr.**
  **D-67316 Carlsberg (DE)**
- **Klimesch, Roger, Dr.**
  **D-64665 Alsbach-Haehnlein (DE)**

(56) Entgegenhaltungen:
**EP-A- 0 200 351**
**EP-A- 0 384 264**
**EP-A- 0 571 882**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 608 707 B1

## Beschreibung

Die vorliegende Erfindung betrifft Cooligomere aus α-Olefinen mit 3 bis 12 C-Atomen und 0,01 bis 5 mol-% Ethylen, erhältlich durch Umsetzung von α-Olefinen mit 3 bis 12 C-Atomen und Ethylen in Gegenwart von Katalysatorsystemen, die als aktive Bestandteile Metallocenkomplexe der allgemeinen Formel I

$$Cp_2MX_2 \qquad \text{I},$$

in der Cp eine unsubstituierte Cyclopentadienyl-Einheit und/oder eine Mono-$C_1$- bis $C_4$-alkylcyclopentadienyl-Einheit, M ein Zirkonium- oder Hafniumatom ist und in der die Liganden X für Hydrid- und/oder Halogenanionen und/oder eine Methylgruppe stehen,
und
offenkettige oder cyclische Alumoxanverbindungen der allgemeinen Formel II oder III

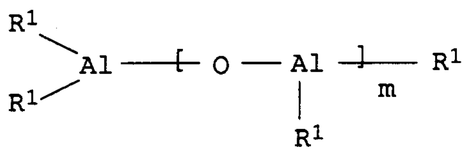

II

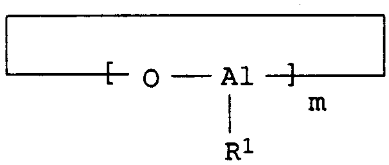

III

wobei $R^1$ eine $C_1$- bis $C_4$-Alkylgruppe bedeutet und m für eine ganze Zahl von 5 bis 30 steht,
enthalten,
wobei die Umsetzung in Suspension ausgeschlossen ist.

Weiterhin betrifft die Erfindung Verfahren zur Herstellung dieser Cooligomeren.

Aus der EP-A 257 696 ist ein Verfahren zur Dimerisierung von α-Olefinen in Gegenwart von Zirkonocenen oder Hafnocenen und Alumoxan bekannt.

Darüber hinaus sind Verfahren bekannt, bei denen Mischungen von α-Olefinen unter Einsatz von Metallocen-Katalysatoren zu Cooligomeren verknüpft werden.

So beschreiben die EP-A 200 351 und EP-A 295 026 die Herstellung von flüssigen Ethylen-Randomcopolymeren mit einem Gehalt von 10 bis 85 mol-% Ethylen.

Unter Verwendung kationischer Metallocenkomplexe lassen sich ebenfalls Cooligomere darstellen, wie in der EP-A 443 686 beschrieben wird.

Aus der EP-A 571 882 sind Verfahren zur Herstellung von Polyolefinwachsen in Suspension bekannt.

Die EP-A 384 264 beschreibt Polypropylenwachse, die kristallin sind und durch Schmelzeigenschaften sowie hohe Tropfpunkte charakterisiert sind.

Derartige Cooligomere stellen gängige Ausgangsstoffe für die Synthese von Schmierstoffen, Kraftstoff- und Öladditiven oder Weichmachern dar.

Diese Verfahren zur Oligomerisierung von Olefinen weisen jedoch den Nachteil auf, daß mit ihnen nur geringe Umsätze an Olefinen erzielt werden können. Vor diesem Hintergrund beschreibt die EP-A 268 214 ein Verfahren, bei dem es gelingt, die Produktivität durch Zusatz von Wasserstoff zu erhohen. Dieses Verfahren weist jedoch den Nachteil auf, daß es auf schwer zugänglichen Übergangsmetallkatalysatoren der Formel $(R_5C_5)_2MX_2$ beschränkt ist. Unsubstituierte Verbindungen der Formel $(Cp)_2MX_2$ führen dagegen zu einer Hydrierung der endständigen Doppelbindungen der Oligomeren. Darüber hinaus ist der produktivitätserhöhende Einfluß des Wasserstoffs gering.

Aufgabe der vorliegenden Erfindung war es daher, lineare Cooligomere von α-Olefinen zur Verfügung zu stellen, die einen hohen Gehalt an endständigen Doppelbindungen aufweisen und die mit hoher Produktivität unter Verwendung leicht zugänglicher Katalysatorsysteme hergestellt werden können.

Demgemäß wurden die eingangs definierten Cooligomere gefunden.

Außerdem wurden Verfahren zur Herstellung dieser Cooligomeren gefunden.

Von den α-Olefinen mit 3 bis 12 C-Atomen sind Verbindungen mit 3 bis 8 C-Atomen, also Propylen, 1-Buten, 1-Propen, 1-Hexen, 1-Hepten und 1-Octen sowie 4-Methylpenten-1 oder Vinylcyclohexan bevorzugt.

Die Menge an Ethylen in den erfindungsgemäßen Cooligomeren beträgt 0,01 bis 5 mol-%, bevorzugt 0,01 bis 3

mol-%, insbesondere 0,01 bis 2 mol-%.

Bei den Metallocenkomplexen der allgemeinen Formel I

$$Cp_2MX_2 \qquad \text{I,}$$

handelt es sich um sogenannte Zirkonocene und Hafnocene, mithin um Komplexe des vierwertigen Zirkoniums und Hafniums, bei denen das Metallatom M sandwichartig zwischen zwei unsubstituierte und/oder $C_1$- bis $C_4$-Monoalkyl-substituierte Cyclopentadienyl-Gruppen Cp gebunden ist, wobei die restlichen Valenzen des Zentralatoms M durch Hydrid- und/oder Halogenanionen und/oder durch Methylgruppen abgesättigt sind. Besonders bevorzugt werden solche Zirkonocen- und Hafnocenkatalysatoren verwendet, deren Cyclopentadienyl-Gruppen unsubstituiert sind. Als Halogenanionen können sowohl Fluor-, Chlor-, Brom- und/oder Jodanionen an das Metallatom gebunden sein.

Beispiele für geeignete Metallocenkomplexe sind:

$Cp_2ZrF_2$, $Cp_2ZrCl_2$, $Cp_2ZrBr_2$, $Cp_2ZrJ_2$, $Cp_2ZrHCl$, $Cp_2Zr(CH_3)Cl$, $Cp_2Zr(CH_3)_2$, $Cp_2HfF_2$, $Cp_2HfCl_2$, $Cp_2HfBr_2$, $Cp_2HfJ_2$, $Cp_2HfHCl$, $Cp_2Hf(CH_3)Cl$, $Cp_2Hf(CH_3)_2$.

Zweckmäßigerweise wird nur ein Metallocenkomplex eingesetzt, es ist aber auch möglich, Mischungen verschiedener Metallocenkomplexe zu verwenden. Bevorzugte Liganden X sind Chlorid, Hydrid und die Methylgruppe, als Zentralatom M wird Zirkonium besonders bevorzugt. Insbesondere wird Zirkonocendichlorid der Formel Ia

$$Cp_2ZrCl_2 \qquad \text{Ia}$$

verwendet, dessen Cyclopentadienylgruppen unsubstituiert sind.

Die Metallocenkomplexe der allgemeinen Formel I können auf einfache Weise nach bekannten Verfahren, z.B. nach Brauer (Hrsg.): Handbuch der Präparativen, Anorganischen Chemie, Band 2, 3. Auflage, Seite 1395 bis 1397, Enke, Stuttgart 1978 synthetisiert werden.

Neben den Metallocenkomplexen enthalten die zur Herstellung der erfindungsgemäßen Cooligomeren eingesetzten Katalysatorsysteme noch oligomere organylsubstituierte Aluminiumoxidverbindungen.

Geeignet sind offenkettige oder cyclische Alumoxanverbindung der allgemeinen Formel II oder III

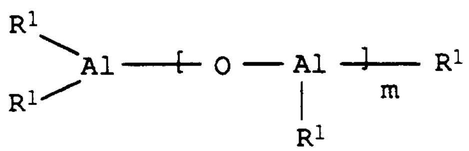

II

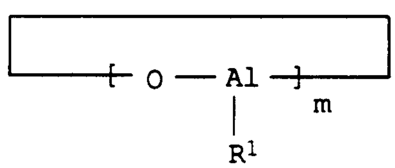

III

wobei $R^1$ eine $C_1$- bis $C_4$-Alkylgruppe, bevorzugt Methyl- oder Ethylgruppe bedeutet und m für eine ganze Zahl von 5 bis 30, bevorzugt 10 bis 25 steht.

Die Herstellung dieser oligomeren Alumoxanverbindungen erfolgt üblicherweise durch Umsetzung einer Lösung von Trialkylaluminium mit Wasser und ist u.a. in der EP-A 284 708 und der US-A 4 794 096 beschrieben.

In der Regel liegen die dabei erhaltenen oligomeren Alumoxanverbindungen als Gemische unterschiedlich langer, sowohl linear als auch cyclischer Kettenmoleküle vor, so daß m als Mittelwert anzusehen ist. Die Alumoxanverbindungen können auch im Gemisch mit anderen Metallalkylen, bevorzugt mit Aluminiumalkylen vorliegen.

Es hat sich als vorteilhaft erwiesen, den Metallocenkomplex und die oligomere Alumoxanverbindung in solchen Mengen zu verwenden, daß das atomare Verhältnis zwischen Aluminium aus der oligomeren Alumoxanverbindung und dem Metallatom M aus dem Metallocenkomplex im Bereich von 10:1 bis $10^6$:1, insbesondere im Bereich von 10:1 bis $10^4$:1 liegt.

Vorzugsweise werden diese Katalysatorsysteme als Lösung eingesetzt. Als Lösungsmittel werden üblicherweise aromatische Kohlenwasserstoffe verwendet, bevorzugt mit 6 bis 20 C-Atomen, insbesondere Xylole oder Toluol sowie deren Mischungen.

Die Herstellung der erfindungsgemäßen Cooligomeren kann in den üblichen, für die Oligomerisation von Olefinen verwendeten Reaktoren entweder diskontinuierlich oder bevorzugt kontinuierlich durchgeführt werden. Geeignete Reaktoren sind u.a. kontinuierlich betriebene Rührkessel, wobei man gegebenenfalls auch eine Reihe von mehreren

hintereinander geschalteten Rührkesseln verwenden kann.

Die Oligomerisation kann in der Gasphase, in flüssigen Monomeren und in inerten Lösungsmitteln durchgeführt werden. Bei der Oligomerisation in Lösungsmitteln werden insbesondere flüssige Kohlenwasserstoffe wie Benzol oder Toluol verwendet.

Bei einem bevorzugten Verfahren zur Herstellung der erfindungsgemäßen Cooligomeren wird zunächst die oligomere Alumoxanverbindung, bevorzugt als Lösung in Toluol, vorgelegt. Hierzu werden die $\alpha$-Olefine mit 3 bis 12 C-Atomen zugegeben und die Temperatur wird erhöht. Anschließend wird Ethylen eingeleitet, wobei die Menge an eingeleitetem Ethylen 0,01 bis 10 mol-%, bevorzugt 0,02 bis 5 mol-%, insbesondere 0,03 bis 3 mol-% beträgt. Nach Zugabe des Metallocenkomplexes wird 5 bis 180 Minuten, bevorzugt 10 bis 90 Minuten oligomerisiert. Die Temperaturen betragen hierbei 0 bis 250°C, bevorzugt 20 bis 200°C, insbesondere 50 bis 180°C und man arbeitet bei Drücken von 1 bis 3000 bar; man kann die Oligomerisation also im Niederdruck-, Mitteldruck- und Hochdruckverfahren durchführen. Die Menge an eingesetztem Katalysator ist nicht kritisch.

Man erhält somit lineare Cooligomere mit Molekulargewichten $\overline{M}_w$ (Gewichtsmittelwert) im Bereich von 100 bis 20000, bevorzugt 100 bis 15000, insbesondere 100 bis 10000, die einen hohen Gehalt an endständigen Doppelbindungen aufweisen und die mit hoher Produktivität unter Verwendung leicht zugänglicher Katalysatorsysteme hergestellt werden können.

## Beispiele

### Cooligomere aus 1-Buten und Ethylen

#### Beispiel 1

In einem 1 l-Rührautoklaven wurden 30 ml einer 1,5 molaren Methylalumoxan-Lösung in Toluol vorgelegt, 500 ml (6,3 mol) flüssiges 1-Buten aufkondensiert und auf 80°C erwärmt. Dabei stellte sich ein Druck von 13 bar ein. Anschließend wurden 0,1 mol Ethylen zugegeben. Nach Zugabe von 28 mg (0,096 mmol) $Cp_2ZrCl_2$ wurde 30 Minuten oligomerisiert.

#### Beispiel 2

In einem 1 l-Rührautoklaven wurden 30 ml einer 1,5 molaren Methylalumoxan-Lösung in Toluol vorgelegt, 500 ml (6,3 mol) flüssiges 1-Buten aufkondensiert und auf 100°C erwärmt. Dabei stellte sich ein Druck von 18 bar ein. Anschließend wurden 0,25 mol Ethylen zugegeben. Nach Zugabe von 25 mg (0,086 mmol) $Cp_2ZrCl_2$ wurde 30 Minuten oligomerisiert.

#### Vergleichsbeispiel V1

Es wurde wie in Beispiel 1 gearbeitet, jedoch ohne Zusatz von Ethylen.

#### Vergleichsbeispiel V2

Es wurde wie in Beispiel 2 gearbeitet, jedoch ohne Zusatz von Ethylen.

Die Ergebnisse und Eigenschaften sind in der Tabelle zusammengestellt.

Die Gewichtsmittelwerte $\overline{M}_w$ und die Zahlenmittelwerte $\overline{M}_n$ wurden durch GPC bestimmt.

Die Menge an Ethylen in den Cooligomeren wurde durch NMR bestimmt, der Gehalt an endständigen Doppelbindungen durch Infrarotspektroskopie.

Tabelle

| Beispiel | Atomverhältnis Al:Zr | Ausbeute [g] | Produktivität des Katalysatorsystems [ml Produkt/g Katalysatorsystem x Stunde] | $\bar{M}_w$ | $\bar{M}_w:\bar{M}_n$ | Menge an Ethylen im Produkt [mol-%] | Gehalt an endständigen Doppelbindungen [%] |
|---|---|---|---|---|---|---|---|
| 1 | 470:1 | 430 | 30 700 | 1900 | 1,95 | 1,2 | > 99 |
| 2 | 520:1 | 470 | 37 600 | 4000 | 4,25 | 2,8 | > 99 |
| V1 | 470:1 | 51 | 3 600 | 1400 | 1,90 | - | 98 |
| V2 | 520:1 | 71 | 5 680 | 1350 | 2,0 | - | 98 |

## Patentansprüche

1. Cooligomere aus α-Olefinen mit 3 bis 12 C-Atomen und 0,01 bis 5 mol-% Ethylen, erhältlich durch Umsetzung von α-Olefinen mit 3 bis 12 C-Atomen und Ethylen in Gegenwart von Katalysatorsystemen, die als aktive Bestandteile Metallocenkomplexe der allgemeinen Formel I

$$Cp_2MX_2 \qquad \text{I,}$$

in der Cp eine unsubstituierte Cyclopentadienyl-Einheit und/oder eine Mono-$C_1$- bis $C_4$-alkylcyclopentadienyl-Einheit, M ein Zirkonium- oder Hafniumatom ist und in der die Liganden X für Hydrid- und/oder Halogenanionen und/oder eine Methylgruppe stehen,
und
offenkettige oder cyclische Alumoxanverbindungen der allgemeinen Formel II oder III

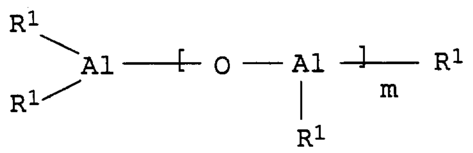

II

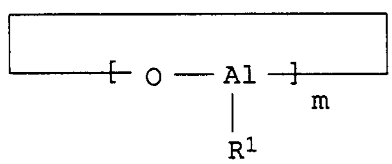

III

wobei $R^1$ eine $C_1$- bis $C_4$-Alkylgruppe bedeutet und m für eine ganze Zahl von 5 bis 30 steht,
enthalten,
wobei die Umsetzung in Suspension ausgeschlossen ist.

2. Cooligomere nach Anspruch 1, dadurch gekennzeichnet, daß sie 0,01 bis 3 mol-% Ethylen enthalten.

3. Verfahren zur Herstellung von Cooligomeren gemäß den in Anspruch 1 definierten Verfahrensbedingungen.

4. Verfahren zur Herstellung von Cooligomeren nach Anspruch 3, dadurch gekennzeichnet, daß man die Umsetzung bei Temperaturen von 0 bis 250°C und bei Drücken von 1 bis 3000 bar durchführt.

## Claims

1. A cooligomer of an α-olefin of 3 to 12 carbon atoms and from 0.01 to 5 mol% of ethylene, obtainable by reacting an α-olefin of 3 to 12 carbon atoms and ethylene in the presence of a catalyst system which contains, as active components, a metallocene complex of the formula I

$$Cp_2MX_2 \qquad \text{I,}$$

where Cp is an unsubstituted cyclopentadienyl unit and/or a mono-$C_1$-$C_4$-alkylcyclopentadienyl unit, M is a zirconium or hafnium atom and the ligands X are each hydride and/or halogen anions and/or a methyl group
and
an open-chain or cyclic alumoxane compound of the formula II or III

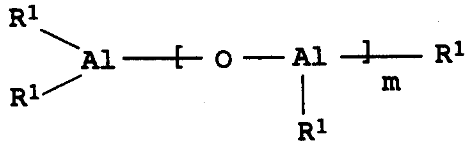

II

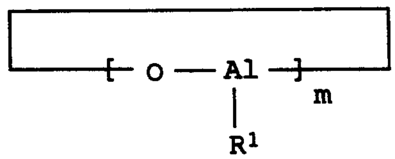

III

where $R^1$ is $C_1$-$C_4$-alkyl and m is an integer of from 5 to 30,
with the exclusion of a reaction in suspension.

2. A cooligomer as claimed in claim 1, which contains from 0.01 to 3 mol% of ethylene.

3. A process for the preparation of a cooligomer in accordance with the process conditions defined in claim 1.

4. A process for the preparation of a cooligomer as claimed in claim 3, wherein the reaction is carried out at from 0 to 250°C and at from 1 to 3000 bar.

## Revendications

1. Co-oligomères d'α-oléfines avec 3 à 12 atomes de carbone et 0,01 à 5 % molaires d'éthylène, pouvant être obtenus par conversion d'α-oléfines avec 3 à 12 atomes de carbone et d'éthyléne en présence de systèmes de catalyseur qui contiennent à titre de constituants actifs des complexes de métallocènes répondant à la formule générale I

$$Cp_2MX_2 \qquad I$$

dans laquelle Cp est une unité cyclopentadiényle non-substituée et/ou une unité mono-(alkyle en $C_1$ à $C_4$)-cyclopentadiényle, M est un atome de zirconium ou d'hafnium et dans laquelle les ligands X représentent des anions d'hydrure et/ou d'halogène et/ou un groupe méthyle,
et
des composés alumoxanes à chaîne ouverte ou cycliques répondant à la formule générale II ou III

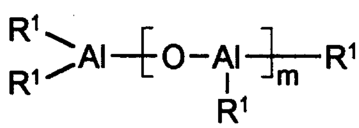

II

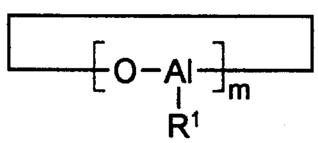

III

où $R^1$ représente un groupe alkyle en $C_1$ à $C_4$ et m représente un nombre entier de 5 à 30,
la conversion en suspension étant exclue.

2. Co-oligomères selon la revendication 1, caractérisés en ce qu'ils contiennent 0,01 à 3 % molaires d'éthylène.

3. Procédé de préparation de co-oligomères conformément aux conditions du procédé définies à la revendication 1.

4. Procédé de préparation de co-oligomères selon la revendication 3, caractérisé en ce que la conversion est effectuée à des températures de 0 à 250 °C et à des pressions de 1 à 3.000 bar.